# EUROPEAN PATENT APPLICATION

(11) **EP 4 527 386 A1**
(43) Date of publication of application: **26.03.2025**
(21) Application number: 23198443.6
(22) Date of filing: 20.09.2023
(51) Int. Cl.: A61K 31/4184, A61K 31/44, A61K 31/519, A61K 31/635, A61K 45/06, A61P 35/00, A61K 38/00

(54) **ANTI-CANCER PHARMACEUTICAL COMPOSITION COMPRISING REGORAFENIB**

(71) Applicant: Eberhard-Karls-Universität Tübingen Universitätsklinikum, 72076 Tübingen BW Baden-Württemberg (DE)
(72) Inventor: TABATABAI, Ghazaleh, 72076 Tübingen (DE); HAEUSSER, Lara Annina, 72076 Tübingen (DE); KUHLBURGER, Laurence, 72070 Tübingen (DE); MERK, Daniel Josef Wolfgang, 71134 Aidlingen (DE)
(74) Representative: Witte, Weller & Partner Patentanwälte mbB

(57) **Abstract**

The present invention relates to a pharmaceutical composition for the treatment and/or prophylaxis of cancer comprising regorafenib and an additional active agent, and to regorafenib in combination with an additional active agent, to a method for the treatment and/or prophylaxis of cancer comprising regorafenib and an additional active agent, and to a method for the preparation of a pharmaceutical composition for the treatment and/or prophylaxis of cancer comprising the formulation of regorafenib and an additional active agent into a pharmaceutically acceptable carrier.

## Description

The present invention relates to a pharmaceutical composition for the treatment and/or prophylaxis of cancer comprising regorafenib and an additional active agent, and to regorafenib in combination with an additional active agent, to a method for the treatment and/or prophylaxis of cancer comprising regorafenib and an additional active agent, and to a method for the preparation of a pharmaceutical composition for the treatment and/or prophylaxis of cancer comprising the formulation of regorafenib and an additional active agent into a pharmaceutically acceptable carrier.

### FIELD OF THE INVENTION

The present invention relates to the field of drug development, more particular the field of anti-cancer pharmaceutical compositions.

### BACKGROUND OF THE INVENTION

Brain tumors are one of the major challenges to current drug development. This applies in particular to drugs for the treatment of so-called glioblastomas.

Glioblastoma are incurable primary tumors of the central nervous system. The standard of care for newly diagnosed glioblastoma outside clinical trials includes a combination of radiation therapy, alkylating chemotherapy and tumor-treating fields. Yet, for progressive glioblastoma, only lomustine is registered in Europe.

The REGOMA trial had investigated regorafenib versus lomustine in a phase 2-trial; see Lombardi et al., Regorafenib compared with lomustine in patients with relapsed glioblastoma (REGOMA): a multicentre, open-label, randomised, controlled, phase 2 trial; Lancet Oncol. 2019 Jan;20(1):110-119.

Furthermore, the use of regorafenib in a compassionate-use-program was recently reported; see Tzaridis et al., Regorafenib in advanced high-grade glioma: a retrospective bicentric analysis; Neuro Oncol. 2019 Jul 11;21(7):954-955.

Yet, the regorafenib arm in the Glioblastoma Adaptive Global Innovative Learning Environment (GBM Agile) master protocol was stopped after an interim analysis that showed a low probability of improvement of overall survival compared with control.

Nevertheless, there are considerations and still the desire to be able to apply regorafenib therapeutically, in order to have invested the development and research efforts to date in a meaningful way. However, so far there are no ideas or approaches in the state of the art in this regard.

The object underlying the invention is therefore to provide a pharmaceutical composition based on regorafenib or an improved way of use of regorafenib that avoids the disadvantages described in the prior art. In particular, such a pharmaceutical composition or use of regorafenib is to be provided which exhibits a high level of efficacy against tumors, in particular brain tumors such as glioblastoma.

### SUMMARY OF THE INVENTION

The object underlying the invention is achieved by the provision of a pharmaceutical composition for the treatment and/or prophylaxis of cancer comprising regorafenib and at least one 'modulator of response to regorafenib' and a pharmaceutically acceptable carrier.

The object underlying the invention is also achieved by the provision of regorafenib in combination with at least one 'modulator of response to regorafenib' for use in the treatment and/or prophylaxis of cancer.

The following explanations, features, advantages and embodiments apply to both the pharmaceutical composition according to the invention and the combination of regorafenib and 'modulator of response to regorafenib' according to the invention.

The inventors have used a functional genomics-based approach to discover and validate potential 'modulators of response to regorafenib' treatment in experimental tumors, in particular glioma, *in vitro, in vivo* and ex *vivo.*

The inventors have discovered and validated, by using CRISPR/Cas9 activation and knockout screens during treatment of various cell lines with regorafenib along with animal models, active agents referred to as 'modulator of response to regorafenib'. Said modulators modulate, preferably increase the anti-cancer activity of regorafenib compared with regorafenib administration alone in a monotherapy setting. The modulators can be active ingredients or agents of any kind, preferably small molecule compounds with proven target inhibitory properties.

The inventors have found that administration of a combination of regorafenib and the modulator (combination therapy) shows significantly improved efficacy against tumors, in particular gliomas, compared to the respective single administration of regorafenib and/or modulator (monotherapy).

Also included according to the invention are the solvates and the solvates of the salts of regorafenib and of the 'modulator of response to regorafenib', respectively.

Salts preferred for the purposes of the present invention are physiologically or pharmaceutically acceptable salts of regorafenib and of the 'modulator of response to regorafenib', respectively. Also encompassed, however, are salts which are themselves not suitable for pharmaceutical applications but can be used for example for the isolation or purification of the regorafenib and of the 'modulator of response to regorafenib', respectively.

Examples of pharmaceutically acceptable salts of the regorafenib and of the 'modulator of response to regorafenib' include salts of inorganic bases like ammonium salts, alkali metal salts, in particular sodium or potassium salts, alkaline earth metal salts, in particular magnesium or calcium salts; salts of organic bases, in particular salts derived from cyclohexylamine, benzylamine, octylamine, ethanolamine, diethanolamine, diethylamine, triethylamine, ethylenediamine, procaine, morpholine, pyrroline, piperidine, N-ethylpiperidine, N-methylmorpholine, piperazine as the organic base; or salts with basic amino acids, in particular lysine, arginine, ornithine and histidine.

Examples of pharmaceutically acceptable salts of the regorafenib and of the 'modulator of response to regorafenib' also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, toluenesulfonates or benzenesulfonates; or salts with acidic amino acids, in particular aspartate or glutamate.

Solvates for the purposes of the invention refer to those forms of the regorafenib and of the 'modulator of response to regorafenib', which in the solid or liquid state form a complex by coordination with solvent molecules. Hydrates are a specific form of solvates in which the coordination takes place with water.

The regorafenib and the 'modulator of response to regorafenib' may also be complexed, e.g., with iron, calcium, etc., in which the compound may act as a ligand, so that a corresponding complex is also subject of the present invention.

According to the invention, "prophylaxis" and "treatment" of cancer refer to any measure to prevent or mitigate or ameliorate the disease or its manifestation or development.

"Pharmaceutically acceptably carriers" are well known to the skilled person. They allow a proper formulation of the compounds or active agents according to the invention, i.e., regorafenib and of the 'modulator of response to regorafenib', and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminum hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

In an embodiment of the invention the combination of regorafenib and the 'modulator of response to regorafenib' results in a synergistic anti-cancer effect.

In their experiments, the inventors were able to demonstrate that the interaction of regorafenib and the modulator leads not only to a merely additive effect of the two active ingredients/agents, but to a synergistic effect of the active ingredients. This effect ensures a particularly potent anti-cancer effect of the pharmaceutical composition according to the invention.

In an embodiment of the invention said 'modulator of response to regorafenib' is a BCL2 inhibitor, preferably selected from the group consisting of navitoclax and venetoclax.

In yet another embodiment of the invention said 'modulator of response to regorafenib' is a MEK1/2 inhibitor, preferably selected from the group consisting of selumetinib and trametinib.

In still another embodiment of the invention said 'modulator of response to regorafenib' is a αᵥ integrin inhibitor, preferably cilengitide.

In a further embodiment of the invention said 'modulator of response to regorafenib' is an ErbB inhibitor, preferably sapitinib.

In still another embodiment of the invention said 'modulator of response to regorafenib' is an ARAF inhibitor, preferably sorafenib.

In yet another embodiment of the invention said 'modulator of response to regorafenib' is a calcium channel blocker, preferably bepridil hydrochloride.

In still another embodiment of the invention said 'modulator of response to regorafenib' is a phospholipase inhibitor, preferably U73122.

These measures according to the invention have the advantage that active substances are already provided as modulators which have been shown to lead to an enhancement of the anti-cancer activity of regorafenib in the sense of synergism in the well-established and recognized experimental models used by the inventors. They are therefore particularly suitable according to the invention.

In another embodiment the cancer is a brain tumor, preferably a glioblastoma.

With this measure, the pharmaceutical composition or combination according to the invention are adapted to the treatment of such tumors or cancer entities for which only few or hardly any pharmaceutical intervention options are currently available. The invention therefore advantageously satisfies a currently particularly urgent medical need.

Another subject-matter of the invention relates to a method for the treatment and/or prophylaxis of cancer comprising the pharmaceutical composition and/or the combination according to the invention to an individual in need, preferably a human individual.

A yet further subject-matter of the invention relates to a method for the preparation of a pharmaceutical composition for the treatment of cancer comprising the formulation of regorafenib and at least one 'modulator of response to regorafenib' into a pharmaceutically acceptable carrier.

The features, properties, advantages and embodiments of the pharmaceutical composition according to the invention and the combination according to the invention apply equally to the above methods according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

Reference is made to figures in which the following is shown.

### BRIEF DESCRIPTION OF THE FIGURES

- Fig. 1:: CRISPR screen analyses using an activation library in GS9 (A), LN229 (B), LN18 (C), LNZ308 (D), and T98G cells (E) to identify modulators of re-sponse to regorafenib.
- Fig. 2:: CRISPR screen analyses using a knockout library in GS9 (A) and LN229 (B) to identify modulators of response to regorafenib.
- Fig. 3:: Heatmaps for an overview of all experimentally tested combinations based on regorafenib CRISPR/Cas9 activation (A) and knockout (B) screens.
- Fig. 4:: Acute cytotoxicity synergy assay results (A, C) and clonogenic survival assay results (B, D) upon combination of regorafenib with BCL2 inhibitors navitoclax (A, B) or venetoclax (C, D).
- Fig. 5:: Acute cytotoxicity synergy assay results (A, C) and clonogenic survival assay results (B, D) upon combination of regorafenib with MEK1/2 inhibitors selumetinib (A, B) or trametinib (C, D).
- Fig. 6:: Acute cytotoxicity synergy assay result (A) and clonogenic survival assay result (B) upon combination of regorafenib with av integrin inhibitor cilengitide.

### EXAMPLES

The inventors performed CRISPR/Cas9 activation (Calabrese P65-HSF activation library) and knockout (genome-wide knockout library Brunello) screens during treatment with regorafenib, sequenced and analyzed the genomic DNA of the remaining cells. Individual combinations of regorafenib and 'modulators of response to regorafenib' were tested for anti-cancer activity in established murine glioma models *in vivo.*

To identify resistance mechanisms and synthetic lethal interactions of Regorafenib treatment in glioblastoma, the inventors performed genome-wide CRISPR/Cas9 activation and knockout screens under treatment with Regorafenib or DMSO control.

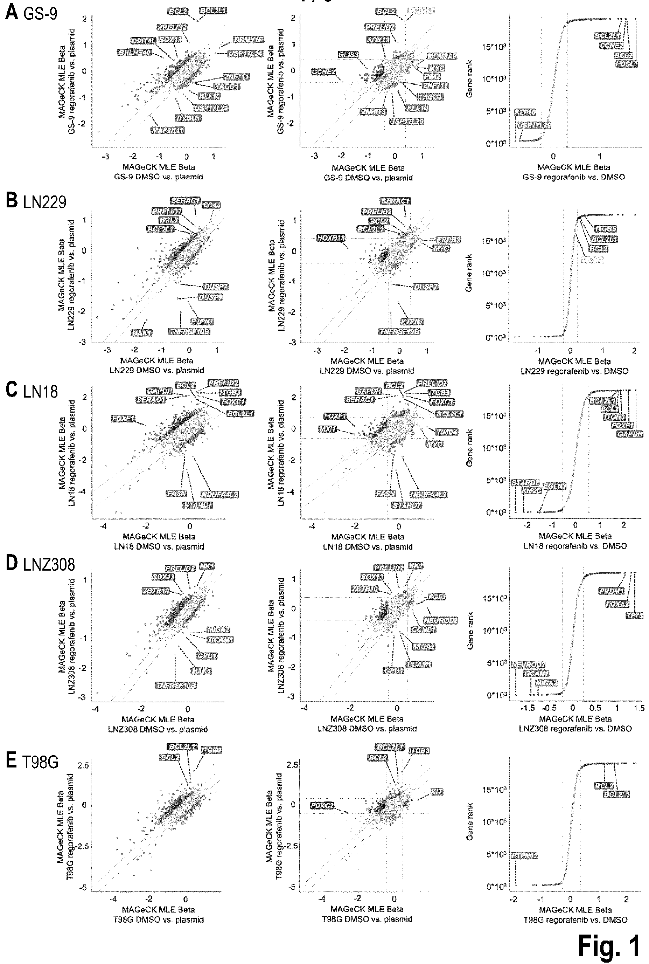

Fig. 1 shows the results of CRISPR screen analyses using an activation library in GS9 (A), LN229 (B), LN18 (C), LNZ308 (D), and T98G cells (E) to identify modulators of response to regorafenib. For A-E: Scatter plot of MAGeCK MLE results from comparing Calabrese sgRNA distributions from DMSO or regorafenib-treated cells to the plasmid library pool (left). The same scatter plot illustrating subcategorization of screen hits according to the 9-squares model (middle). Rankview plot illustrating MAGeCK MLE results from comparing Calabrese sgRNA distributions from regorafenib-treated cells to the corresponding DMSO control (right).

Activation screens (Fig. 1) were performed in five glioma cell lines GS-9, LN229, LN18, LNZ308, and T98G using the Calabrese sgRNA library. Sequencing data was analyzed using MAGeCK MLE. Genes enriched in the Regorafenib treated cells but not in the DMSO treated cells confer resistance against treatment. These genes include members of the Bcl2 family (BCL2, BCL2L1), integrins (ITGB3, ITGB5), and receptor tyrosine kinase ErbB3, for which targeted drugs have been described (Bcl2: venetoclax, navitoclax, obatoclax mesylate; ITGB3, ITGB5: cilengitide; ERBB3: sapitinib).

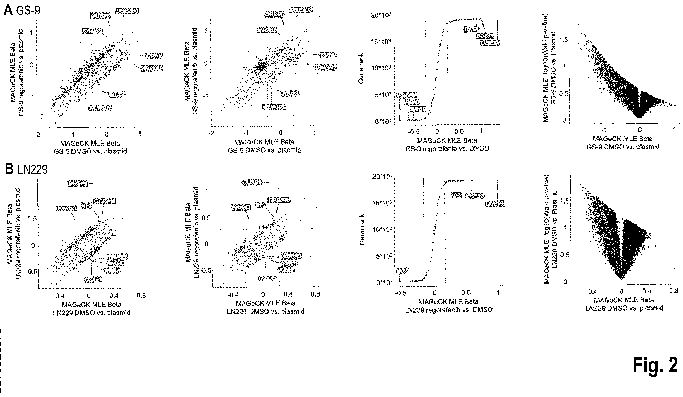

Fig. 2 shows the outcome of CRISPR screen analyses using a knockout library in GS9 (A) and LN229 (B) to identify modulators of response to regorafenib. For A-B: Scatter plot of MAGeCK MLE results from comparing Brunello sgRNA distributions from DMSO or regorafenib-treated cells to the plasmid library pool (left). The same scatter plot illustrating subcategorization of screen hits according to the 9-squares model (middle left). Rankview plot illustrating MAGeCK MLE results from comparing Brunello sgRNA distributions from regorafenib-treated cells to the corresponding DMSO control (middle right). Volcano plot illustrating p- and beta-values of MAGeCK MLE results from DMSO-treated cells compared to plasmid library pool (right).

For the genome-wide CRISPR/Cas9 knockout screens the inventors used Brunello sgRNA library in two glioma cell lines GS-9 and LN229 (Fig. 2). Depleted genes in the regorafenib treated sample which were not affected in the DMSO treated sample are synthetic lethal interactors of the treatment. Among those depleted genes the inventors identified intracellular kinases (ARAF, MAP2K1), a calcium channel subunit (CACNG7), and phospholipase C family members (PLCE1, PLCG1), which can be targeted by known drugs (ARAF: sorafenib, tovorafenib, LY-3009120; MAP2K1: selumetinib, avutometinib, binimetinib, pimasertib, trametinib; CACNG7: bepridil hydrochloride; PLCE1, PLCG1: U73122).

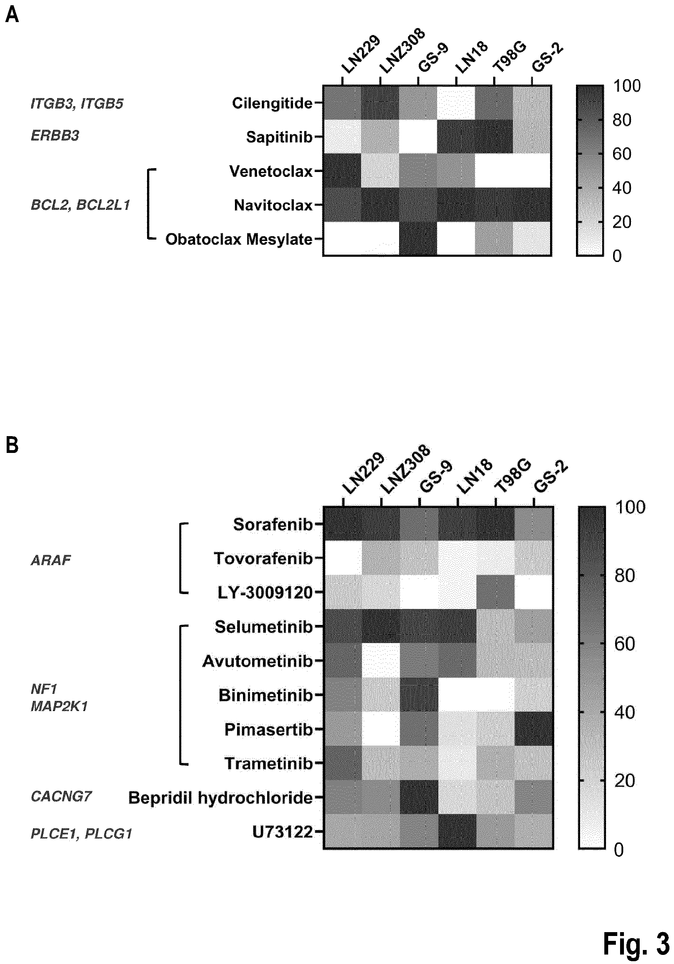

Fig. 3 shows heatmaps for an overview of all experimentally tested combinations based on regorafenib CRISPR/Cas9 activation (A) and knockout (B) screens. In the heatmaps, the intensity of the red color or grey/black stands for the synergism of the drugs mentioned per row with regorafenib in the different cell lines (columns). The targets from the CRISPR/Cas9 screens, which make up the respective groups, are again shown to the left.

The combined inhibition of a resistance mechanism or a synthetic lethal interactor with regorafenib treatment should enhance the effect of regorafenib alone. Therefore, drugs targeting the different identified gene products were used in acute cytotoxicity synergy assays in combination with regorafenib to analyze the synergistic effect of drug combinations. Each of the 15 drugs was tested in a 72-hour acute cytotoxicity synergy assay in combination with regorafenib on six glioma cell lines in two independent runs. The calculated ZIP synergy score for each combination indicates if a combination is synergistic, the higher the score the stronger is the synergism. For the heatmaps shown in Fig. 3 the synergy scores were normalized within each cell line taking the highest synergy score detected in this cell line as 100% and a synergy score of 0 as 0%. For each target shown here identified in the CRISPR/Cas9 screens, the inventors detected a synergistic effect of the drug combination (for at least one drug when several drugs for the same target were tested).

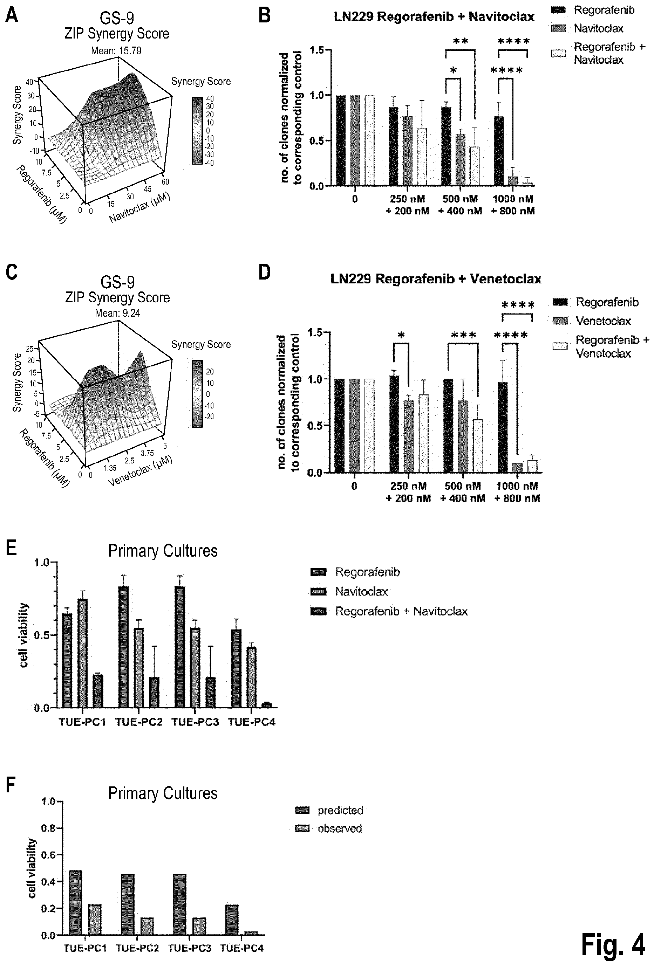

Fig. 4 shows the results of the acute cytotoxicity synergy assay (A, C) and clonogenic survival assay (B, D) upon combination of regorafenib with BCL2 inhibitors navitoclax (A, B) or venetoclax (C, D). Acute cytotoxicity assay results of four glioblastoma primary cultures treated with regorafenib and navitoclax (E, F). Cell viability of technical triplicates for each primary culture (E). Predicted and observed drug combination response following Bliss independence model (F).

For drugs targeting Bcl2 family members (navitoclax, venetoclax), MEK1/MAP2K1 (selumetinib, trametinib), integrins (cilengitide), and ARAF (sorafenib) further assays were performed to analyze the synergistic effect in combination with Regorafenib. Navitoclax and venetoclax show synergistic effects with Regorafenib in acute cytotoxicity synergy assays (Figs. 4 A and C, only one out of six cell lines shown) as well as significantly reduced numbers of clones in clonogenic survival assays comparing the combination treatment with regorafenib monotherapy (Figs. 4 B and D, only one out of four cell lines shown). The inventors extracted primary cells from glioblastoma tissue to test our drug combinations ex *vivo.* Analyzing the combination effect on cell viability of regorafenib and navitoclax on four glioblastoma primary cultures the observed effect of the combination treatment was in each model synergistic compared to the predicted effect calculated from both monotherapies (Figs. 4 E and F). The combination of navitoclax and regorafenib was further validated in vivo in VM/Dk mice intracranially transplanted with SMA560 cells (data not shown). The mice were treated either with the drug combination, regorafenib, or navitoclax monotherapies, or a solvent control for three weeks. Time until onset of neurological symptoms was observed. The solvent control group reached its median after 58 days, navitoclax monotherapy after 60 days, and regorafenib monotherapy after 67 days. The regorafenib and navitoclax combination treatment group did not reach the median until day 120 when the experiment had to be stopped due to legal regulation. The combination showed a significant prolonged time until onset of neurological symptoms *in vivo.*

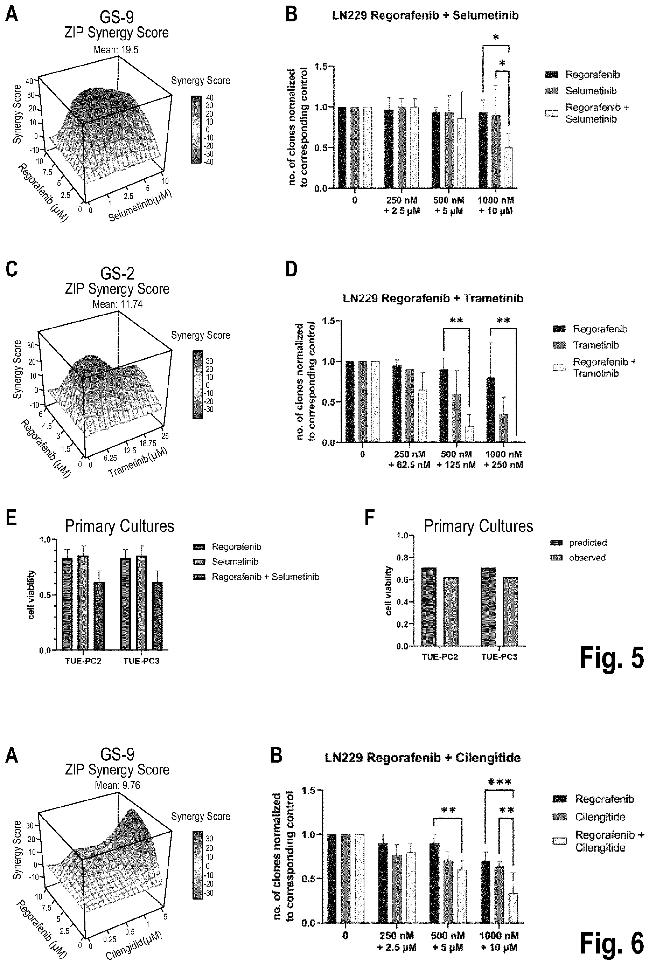

Fig. 5 shows the results of the acute cytotoxicity synergy assay (A, C) and clonogenic survival assay (B, D) upon combination of regorafenib with MEK1/2 inhibitors selumetinib (A, B) or trametinib (C, D). Acute cytotoxicity assay results of two glioblastoma primary cultures treated with regorafenib and selumetinib (E, F). Cell viability of technical triplicates for each primary culture (E). Predicted and observed drug combination response following Bliss independence model (F).

MEK1/2 inhibition in combination with regorafenib was also analyzed in more detail (Fig. 5). The acute cytotoxicity synergy assays with regorafenib and selumetinib or regorafenib and trametinib show synergistic synergy scores (Figs. 5 A and C, only one out of six cell lines shown). Clonogenic survival assays with each combination show significantly reduced number of clones when combining regorafenib and selumetinib compared to both monotherapies. The combination of regorafenib and trametinib leads to significantly reduced clonogenic survival compared to regorafenib treatment (Figs. 5 B and D, only one out of four cell lines shown). The effect of the combination was also tested ex *vivo* in two glioblastoma primary cultures and showed a stronger observed effect compared to the predicted effect calculated from both monotherapies (Figs. 5 E and F).

Fig. 6 shows the results of the acute cytotoxicity synergy assay (A) and clonogenic survival assay (B) upon combination of regorafenib with αᵥ integrin inhibitor cilengitide.

The CRISPR/Cas9 screen identified ITGB3 and ITGB5 as resistance genes for regorafenib therapy. Therefore, the inventors expect a synergistic effect when combining regorafenib with αᵥ integrin inhibitor cilengitide. The synergism was detected in acute cytotoxicity synergy assays (Fig. 6 A, only one out of five cell lines shown) and also in clonogenic survival assays in which the combination of regorafenib and cilengitide is significantly reduced compared to both monotherapies (Fig. 6 B, only one out of four cell lines shown).

The inventors discovered functionally instructed molecular hits, designed a drug library for further validation by functional assays *in vitro* and combination treatments in well-established and accepted animal models *in vivo.* Taken together, the inventors discovered and validated functionally instructed combination treatment options involving regorafenib.

## Claims

1. A pharmaceutical composition for the treatment and/or prophylaxis of cancer comprising regorafenib and at least one 'modulator of response to regorafenib' and a pharmaceutically acceptable carrier.

2. The pharmaceutical composition of claim 1, wherein the combination of regorafenib and the 'modulator of response to regorafenib' results in a synergistic anti-cancer effect.

3. The pharmaceutical composition of claim 1 or 2, wherein the 'modulator of response to regorafenib' is a BCL2 inhibitor, preferably selected from the group consisting of navitoclax and venetoclax.

4. The pharmaceutical composition of claim 1 or 2, wherein the 'modulator of response to regorafenib' is a MEK1/2 inhibitor, preferably selected from the group consisting of selumetinib and trametinib.

5. The pharmaceutical composition of claim 1 or 2, wherein the 'modulator of response to regorafenib' is a αᵥ integrin inhibitor, preferably cilengitide.

6. The pharmaceutical composition of claim 1 or 2, wherein the 'modulator of response to regorafenib' is an ErbB inhibitor, preferably sapitinib.

7. The pharmaceutical composition of claim 1 or 2, wherein the 'modulator of response to regorafenib' is an ARAF inhibitor, preferably sorafenib.

8. The pharmaceutical composition of claim 1 or 2, wherein the 'modulator of response to regorafenib' is a calcium channel blocker, preferably bepridil hydrochloride.

9. The pharmaceutical composition of claim 1 or 2, wherein the 'modulator of response to regorafenib' is a phospholipase inhibitor, preferably U73122.

10. The pharmaceutical composition of any of claims 1-9, wherein the cancer is a brain tumor, preferably a glioblastoma.

11. Regorafenib in combination with at least one 'modulator of response to regorafenib' for use in the treatment and/or prophylaxis of cancer.

12. A method for the treatment and/or prophylaxis of cancer comprising the pharmaceutical composition of any of claims 1-10 and/or the combination of claim 11 to an individual in need, preferably a human individual.

13. A method for the preparation of a pharmaceutical composition for the treatment and/or prophylaxis of cancer comprising the formulation of regorafenib and at least one 'modulator of response to regorafenib' into a pharmaceutically acceptable carrier.
